(19) Europäisches Patentamt

European Patent Office

Office européen des brevets

(11) **EP 1 259 287 B1**

(12) **EUROPÄISCHE PATENTSCHRIFT**

(45) Veröffentlichungstag und Bekanntmachung des Hinweises auf die Patenterteilung:
**26.01.2005   Patentblatt 2005/04**

(21) Anmeldenummer: 01905796.7

(22) Anmeldetag: **19.02.2001**

(51) Int Cl.$^7$: **A61N 1/365**, A61N 1/39

(86) Internationale Anmeldenummer:
**PCT/EP2001/001827**

(87) Internationale Veröffentlichungsnummer:
**WO 2001/060451 (23.08.2001 Gazette 2001/34)**

(54) **VORRICHTUNG ZUR DETEKTION TACHYKARDER HERZRHYTHMUSSTÖRUNGEN**

DEVICE FOR DETECTING TACHYCARDIAC RHYTHM DISTURBANCES

DISPOSITIF DE DETECTION DE PERTURBATIONS TACHYCARDIQUES DU RYTHME CARDIAQUE

(84) Benannte Vertragsstaaten:
**CH DE FR GB IT LI NL**

(30) Priorität: **17.02.2000   DE 10008324**

(43) Veröffentlichungstag der Anmeldung:
**27.11.2002   Patentblatt 2002/48**

(73) Patentinhaber: **BIOTRONIK Mess- und Therapiegeräte GmbH & Co Ingenieurbüro Berlin 12359 Berlin (DE)**

(72) Erfinder: **KAYE, Gerry, C.**
**Kirk Ella, Hull HU10 7QL (GB)**

(74) Vertreter: **Eisenführ, Speiser & Partner Patentanwälte Rechtsanwälte Spreepalais am Dom Anna-Louisa-Karsch-Strasse 2 10178 Berlin (DE)**

(56) Entgegenhaltungen:
EP-A- 0 793 976          WO-A-98/14240
US-A- 4 303 075          US-A- 5 179 946
US-A- 5 385 576

**Beschreibung**

Die Erfindung betrifft eine Vorrichtung zur Detektion tachykarder Herzrhythmusstörungen, mit einer Messeinheit zum Aufnehmen und Wiedergeben eines von der intrakardialen Impedanz abhängigen Messsignals an ihrem Ausgang und einer eingangsseitig mit der Messeinheit verbundenen Auswerteeinheit.

Weiterhin betrifft die Erfindung eine implantierbare Elektrostimulationsvorrichtung zur Behandlung tachykarder Herzrhythmusstörungen, mit einer Detektionseinheit, einer Steuereinheit und einer Therapieeinheit, die für die Erzeugung einer an das Herz zu übertragenden Kardioversions- oder Defibrillationselektrotherapie ausgebildet ist, wobei die Detektionseinheit eine Messeinheit zum Aufnehmen und Wiedergeben eines der intrakardialen Impedanz entsprechenden Messsignals an ihrem Ausgang und eine eingangsseitig mit der Messeinheit verbundene Auswerteeinheit aufweist und die Steuereinheit Ausgangssignale der Auswerteeinheit empfängt und die Tätigkeit der Messeinheit, der Auswerteeinheit und der Therapieeinheit steuert.

Aufgrund des geringeren spezifischen Widerstandes von Blut im Vergleich mit dem Myokardgewebe variiert die intrakardiale Impedanz mit dem im Verlaufe des Herzzyklus wechselnden Blutvolumen in den Herzkammern. Somit kann durch Auswertung einer intrakardialen Impedanzmessung die Pumptätigkeit des Herzens überwacht und aus Veränderungen des Amplitudenverlaufs oder der Frequenz des periodischen Impedanzsignals auf das Vorliegen von Herzrhythmusstörungen wie Tachykardie oder Fibrillation geschlossen werden.

Eine solche Überwachungsvorrichtung ist aus der EP 0009 255 A1 bekannt. Diese Druckschrift offenbart einen implantierbaren Defibrillator mit zwei Messelektroden, die am distalen Ende eines in den rechten Ventrikel eingeführten Katheters mit axialem Abstand voneinander angeordnet sind. Eine Steuerlogikeinheit startet eine intrakardiale Impedanzmessung, wenn eine automatische Auswertung eines EKG-Signals auf das mögliche Vorliegen von Fibrillation hinweist. Die Impedanz wird mit Hilfe einer Spannungsmessung zwischen den Elektroden unter einem Wechselstromfluß mit konstanter Modulationsamplitude sowie einer anschließenden Demodulation des Spannungssignals bestimmt. Weist die Amplitude des Impedanzsignals auf eine zu geringe Pumptätigkeit des Herzens hin, wird eine Defibrillationstherapie eingeleitet.

Nachteilig ist bei dieser Vorrichtung, dass die Signale der Meßelektroden aufgrund ihrer Anordnung in einem Ventrikel empfindlich auf Störungen reagieren. So kann schon durch eine körperliche Bewegung ein Kontakt zwischen den Elektroden und dem Myokardgewebe herbeigeführt werden. Dadurch wird jedoch die Messspannung und somit die Impedanzmessung verfälscht. Unnötige und für den Patienten schmerzhafte Defibrillationsschocks können die Folge einer fehlerhaften Impedanzmessung sein.

Aus der US-A-5 427 112 ist es bekannt, zur Erhöhung der Zuverlässigkeit der Detektion von Herzrhythmusstörungen zwei Signale aufzuzeichnen und ihre phasengleiche und an den Herzrhythmus gekoppelte periodische Wiederkehr zu überwachen. Bei dieser bekannten Vorrichtung wird zusätzlich zur Messung des intrakardialen Impedanzsignals seine zeitliche Ableitung bestimmt. Das Impedanzsignal wird in einer Parameterdarstellung als Funktion seiner zeitlichen Ableitung aufgezeichnet. Die so aufgezeichnete Kurve wird mit eingespeicherten Musterkurven verglichen, woraufhin eine Entscheidung über die Notwendigkeit und gegebenenfalls die Art einer Therapie getroffen wird. Dieses Verfahren hat den Nachteil, dass es sehr rechen- und speicheraufwendig ist: es muß zunächst die zeitliche Ableitung des Messsignals bestimmt werden. Messwert samt zeitlicher Ableitung müssen über mindestens die Dauer einer Herzperiode abgespeichert werden. Anschließend muß die Phasenlage der abgespeicherten Daten im Vergleich zu einer voreingespeicherten Musterkurve bestimmt werden, was umfangreiche Rechnungen erfordert. Schließlich muß dann die Differenz der gemessenen Wertepaare und entsprechender Wertepaare der Musterkurve gebildet und letztendlich anhand eines mathematischen Kriteriums bewertet werden.

Die US-A-5 179 946 offenbart einen implantierbaren Defibrillator, bei dem zur Erhöhung der Zuverlässigkeit der Detektion von Herzrhythmusstörungen die intrakardiale Impedanz zwischen zwei an der Außenseite des Herzens befestigten Defibrillationselektroden gemessen wird. Mit Hilfe eines Amplitudendiskriminators wird die ausreichende Pumptätigkeit des Herzens anhand des Impedanzsignals überwacht. Alternativ zur Amplitudendiskrimination wird bei diesem bekannten Defibrillator eine Integration des gemessenen Impedanzsignals durchgeführt. Dieses Vorrichtung ist schalttechnisch aufwendig, weil die Impedanzmessung und die Tachykardietherapie über dieselben Elektroden erfolgen. Zur Vermeidung einer Zerstörung der Messeinheit durch die hohen elektrischen Spannungen, die bei einer Kardioversion oder Defibrillation erzeugt werden, ist daher eine Schutzschaltung erforderlich, die vor der Anwendung der Therapie zwischen die Defibrillationselektroden und die Messeinheit zu schalten ist. Nachteilig ist bei diesem bekannten Defibrillator weiterhin ein für den Patienten belastender, großer operativer Aufwand bei der Implantation der epikardialen Elektroden.

Aufgabe der Erfindung ist es, eine zuverlässige, aber weniger aufwendige Vorrichtung zur Detektion tachykarder Herzrhythmusstörungen sowie eine weniger aufwendige implantierbare Elektrostimulationsvorrichtung zur Behandlung tachykarder Herzrhythmusstörungen anzugeben.

Die Aufgabe wird für eine Vorrichtung zur Detektion tachykarder Herzrhythmusstörungen durch die Merkmale des Anspruchs 1 und für eine implantierbare Elektrostimulationsvorrichtung zur Behandlung tachykarder Herzrhythmusstörungen durch eine Vorrichtung mit den Merkmalen des Anspruchs 20 gelöst.

Grundgedanke der Erfindung ist, die Zuverlässigkeit der Detektion von Herzrhythmusstörungen durch die Bestimmung zweier in ihrem Informationsgehalt über die Herztätigkeit sich ergänzender Signale zu erhöhen, und dabei zugleich aber nur solche Signale heranzuziehen, für deren Bestimmung der Rechen- und Speicherbedarf besonders gering ist.

Dies wird bei der erfindungsgemäßen Vorrichtung zur Detektion tachykarder Herzstörungen ausgehend von den Merkmalen des Oberbegriffs des Anspruchs 1 dadurch erreicht, daß die Auswerteeinheit zusätzlich ausgebildet ist zum Bestimmen und Ausgeben eines zweiten Signals, das vom Integral des Messsignals über mindestens eine definierte zweite Zeitspanne abhängig ist, und des der zweiten Zeitspanne zugeordneten ersten Signals, wobei die zuerst genannte Zeitspanne vor der zweiten Zeitspanne liegt oder mit der zweiten Zeitspanne identisch ist.

Die definierte Zeitspanne kann sich über eine vorbestimmbare Zeitspanne, beispielsweise 5 Sekunden, und/oder über mindestens einen Herzzyklus erstrecken. Die Dauer eines Herzzyklus ist beispielsweise in einem Elektrokardiogramm (EKG) als Zeitspanne zwischen gleichen charakteristischen Punkten aufeinanderfolgender Herzzyklen, etwa den Q-Punkten, bestimmbar.

Die Differenz der Extremwerte des Messsignals innerhalb eines Herzzyklus, also die Peak-to-Peak-Amplitude PPA des Messsignals, zeigt den Unterschied zwischen der intrakardialen Impedanz des systolischen und des diastolischen Herzens an. Während der Systole ist eine minimale Blutmenge im Herzen, wodurch die gemessene Impedanz einen maximalen Wert innerhalb der Herzperiode annimmt. Während der Diastole enthält das Herz eine maximale Blutmenge, die den gemessenen Impedanzwert ein Minimum annehmen läßt. Die PPA ist also ein Maß für die Pumptätigkeit des Herzens. Bei Auftreten einer Tachykardie ist die PPA der intrakardialen Impedanz aufgrund der verringerten Pumpeffizienz des Herzens signifikant verringert. Bei einem fibrillierenden Herzen sinkt die PPA der intrakardialen Impedanz auf etwa Null ab, weil das Blutvolumen des Herzens wegen der unkoordinierten Bewegung der Herzmuskeln kaum veränderlich ist. Insofern ist schon das Absinken der PPA unter einen vorbestimmbaren (patientenabhängigen) Schwellwert ein deutliches Indiz für das Vorliegen einer tachykarden Herzrhythmusstörung.

Die PPA ist in dem Fachmann bekannter Weise einfach und ohne aufwendige Rechnungen sowie ohne Speicherung größerer Datenmengen bestimmbar.

Durch die erfindungsgemäße zusätzliche Bestimmung eines zweiten, vom Integral des Messignals über mindestens eine definierte Zeitspanne abhängigen Signals werden ebenfalls Informationen über die Pumptätigkeit des Herzens gesammelt. Beim Vorliegen einer Tachykardie oder einer Defibrillation nimmt dieses zweite Signal aufgrund der verschlechterten Pumptätigkeit des Herzens andere Werte an als bei normal schlagendem Herzen.

Auch die Integration des Messsignals ist bei durch die Auswerteeinheit der erfindungsgemäßen Vorrichtung in an sich bekannter Weise einfach und ohne Speicheraufwand durchführbar.

Darüber hinaus ist vorteilhaft, daß kurzzeitige Ausschläge des Messsignals eine wesentlich geringere Auswirkung auf das zweite Signal haben als auf die PPA. Solche Ausschläge treten beispielsweise bei einer Lageveränderung von Messelektroden relativ zu den Gefäßwandungen des Herzens auf. Sie können die PPA dadurch verfälschen, daß solch ein zufälliges, ungewöhnlich hohes oder niedriges Messsignal das Maximum oder Minimum während des Messzeitintervalls, unter Umständen auch mehrerer Messzeitintervalle hintereinander bildet. So kann das erste Signal fälschlicherweise auf das Vorliegen einer nicht gegebenen Herzarrhythmie hindeuten. Auf das Integral des Messignals hat solch ein Ausschlag wegen der relativ langen Zeitspanne, über die die Integration durchgeführt wird, jedoch einen nur geringen Einfluß. Das zweite Signal wird in diesem Fall zutreffenderweise also eine normale Herztätigkeit anzeigen. Dadurch wird die unnötige Verabreichung einer Kardioversions- oder Defibrillationstherapie vermieden.

Insofern ergänzen sich die Informationen, die das erste und zweite Signal über die Herztätigkeit liefern und sorgen für eine hohe Zuverlässigkeit der Detektion von Herzrhythmusstörungen durch die erfindungsgemäße Vorrichtung.

Das erste wie das zweite Signal können durch Auswertung des Messsignals innerhalb einer oder mehrerer Zeitspannen bestimmt werden. Beispielsweise ist es möglich, die Auswertung des Messsignals kurzzeitig zu unterbrechen und anschließend fortzusetzen. Möglich ist auch eine Mittelung des über mehrere Zeitspannen bestimmten ersten oder zweiten Signals.

Das erste und das zweite Signal müssen aber nicht immer zugleich bestimmt werden. Es kann beispielsweise auch vorgesehen sein, daß erst, wenn das erste Signal ein Indiz für das Bestehen einer Arrhythmie liefert, die zusätzliche Bestimmung des zweiten Signals in einer nachfolgenden Messung veranlasst wird.

Als Auswertungszeitspanne ist die Dauer eines oder mehrerer Herzzyklen günstig. Die Bestimmung des Start- und des Stopp-Zeitpunktes der Auswertung relativ zum Herzzyklus kann dabei mit an sich bekannten Mitteln erfolgen. Wichtig ist, dass die Vorrichtung alternativ über vorbestimmte, vom Herzzyklus unabhängige Zeitspannen auswerten kann, da beispielsweise bei Fibrillation eine Triggerung relativ zum Herzzyklus nicht funktionieren kann.

Durch die erfindungsgemäßen Maßnahmen wird zum einen sichergestellt, dass eine Elektrostimulations-Therapie bei einem akuten Erfordernis auch angewendet wird. Zugleich wird vermieden, daß eine für den Patienten unangenehme Therapie unnötigerweise allein aufgrund von Schwankungen im Messsignat angewendet wird. Die Auswertung zweier, in ihrer Signifikanz sich ergänzender Messsignale liefert also eine wesentliche Erhöhung der Zuverlässigkeit bei der Detektion von tachykarden Herzrhythmusstörungen. Zum anderen sind beide Messsignale einfach bestimmbar; ihre Auswertung erfordert keinen großen Rechenaufwand. Ein einfacher Vergleich mit Sollwerten oder Sollwertintervallen zeigt schon den aktuellen Zustand des Herzens an.

Bei einer bevorzugten Ausführungsform der Erfindung ist die Impedanzmessung unipolar durchführbar. Bei einer unipolaren Impedanzmessung wird neben der beispielsweise in den rechten Ventrikel eingeführten Messelektrode das implantierte Gehäuse der Vorrichtung zugleich als zweite Elektrode verwendet. Aufgrund des relativ großen Abstandes zwischen diesen Elektroden geht in das unipolare Impedanzsignal nicht nur die Information über das im Herzen befindliche Blutvolumen ein. Vielmehr ist beispielsweise auch Information über die Atemfrequenz im unipolaren Impedanzsignal enthalten. Diese kann separat zur Ermittlung physiologischer Parameter etwa im Rahmen einer Schrittmachertherapie genutzt werden. Soll nur Information über die Pumptätigkeit des Herzens verarbeitet werden, können die niedrigen, durch die Atmung entstehenden Messsignalfrequenzen (max. 1 Hz) leicht vom höherfrequenten Anteil der Herztätigkeit durch entsprechende Filterung des Signals getrennt werden.

Alternativ kann die Impedanzmessung bei der erfindungsgemäßen Vorrichtung auch bipolar durchführbar sein. In diesem Fall wird eine in das Herz eingeführte Messonde zwei elektrisch voneinander isolierte Elektroden aufweisen. Der bei der Messung induzierte Stromfluß erfolgt durch das Blut im Ventrikel. Das Gehäuse der Vorrichtung spielt hier keine Rolle.

In beiden Fällen weist die Messeinheit mindestens zwei Elektroden auf, von denen mindestens eine in eine Herzkammer einführbar ist. Im Fall der unipolaren Messung wird die zweite Elektrode von einem implantierbaren Gehäuse der Vorrichtung gebildet. Im Fall der bipolaren Messung sind beide Elektroden unmittelbar im Bereich des Herzens angeordnet. Die zweite Elektrode kann in diesem Fall außerhalb des Herzens, etwa an der Außenseite des Herzens angeordnet sein, oder ebenfalls in eine Herzkammer eingeführt sein, jedoch nicht notwenigerweise in dieselbe Kammer wie die erste Elektrode. Wesentlich ist, daß der Stromfluss bei der Impedanzmessung durch ein sich im Verlaufe der Herzperiode veränderndes Blutvolumen innerhalb einer oder mehrerer Herzkammern verläuft.

Vorzugsweise ist die intrakardiale Impedanz bei der unipolaren wie bei der bipolaren Impedanzmessung durch Messung einer elektrischen Spannung zwischen den Elektroden bei Beaufschlagung mit einem voreinstellbaren elektrischen Strom bestimmbar. Die Bestimmung der Impedanz erfolgt also anhand des voreingestellten Stromwertes und dem gemessenen Spannungswert nach dem Ohmschen Gesetz. Die Messeinheit weist dementsprechend eine Stromquelle auf. Diese ist mit den Elektroden der Messeinheit derart verbunden, daß sie einen vorbestimmten Messstrom zwischen ihnen erzeugt. Der Messstrom ist vorzugsweise pulsförmig, wie weiter unten näher erläutert wird. Weiterhin weist die Vorrichtung bei diesem Ausführungsbeispiel Spannungsmessmittel, die zum Messen einer elektrischen Spannung zwischen den Elektroden mit diesen verbunden sind. Bei dieser Ausführungsform kann die impedanz durch Division des Messsignals durch die Stromstärke bestimmt werden.

Der für die Impedanzmessung während einer vorbestimmten Zeitspanne T aufgebrachte Energiebetrag E(T) kann bei konstantem Strom I auf einfache Weise aus dem sowieso bestimmten zeitlichen Integral der Spannung U berechnet werden:

$$E(T) = I \times \int_T U(t)\, dt$$

Die so bestimmte Energie kann als zusätzliche Informationsquelle ausgewertet werden.

Alternativ kann bei der erfindungsgemäßen Vorrichtung die intrakardiale Impedanz durch Messung eines Stromflusses zwischen den Elektroden bei Beaufschlagung mit einer voreinstellbaren elektrischen Spannung bestimmbar sein. Hierfür weist die Messeinheit eine Spannungsquelle auf, die mit den Elektroden der Messeinheit derart verbunden ist, daß sie eine vorbestimmte Messspannung zwischen ihnen erzeugt, sowie Strommessmittel, die zum Messen eines elektrischen Stromes zwischen den Elektroden mit diesen verbunden sind.

Zur Vermeidung einer Polarisierung der Elektrode ist in einer Ausführungsform der Erfindung die beaufschlagte, konstante Stromstärke oder Spannung mit einer vorbestimmbaren Zeitabhängigkeit modulierbar derart, daß ein periodischer Wechselstrom fließt bzw. eine periodische Wechselspannung anliegt, deren maximale Stromstärke bzw. dessen maximale Spannungsamplitude in jeder Halbperiode denselben Betrag hat. Pulsform und Frequenz können vorbestimmt werden. Vorzugsweise wird ein Wechselstrom mit einer Rechteck-Pulsform verwendet. In einem Ausführungsbeispiel werden bipolare Rechteckimpulse mit einem Zeitabstand von etwa 50 Millisekunden verwendet. Die positive und die sich unmittelbar daran anschließende negative Halbperiode der Rechteckimpulse haben eine Dauer von je etwa 1 Mikrosekunde.

Aufgrund der unterschiedlichen Frequenzabhängigkeit der Impedanz des Blutes und des Myokardgewebes kann durch geeignete Wahl der Frequenz auch der Impedanzkontrast zwischen Systole und Diastole vergrößert werden, um die Auswertung der PPA noch zuverlässiger hinsichtlich des Nachweises von Herzarrhythmien zu machen. Bei einer Frequenz von 4096 Hz etwa beträgt der spezifische Widerstand von Blut nur ein Drittel des spezifischen Widerstands des Myokardgewebes.

Bei dieser Ausführungsform wird die von der dem Messstrom bzw. der Messspannung eingeprägten Frequenz verursachte Modulation des Messignals vorzugsweise durch eine der zeitlichen Integration vorgeschaltete Demodulationsstufe entfernt. An deren Eingang liegt im Betrieb der Vorrichtung das durch den von der Stromquelle (oder Spannungsquelle) erzeugten Wechselstrom (bzw. Wechselspannung) modulierte Messsignal der Spannungsmessmittel (bzw. Strommessmittel) an. Die Demodulationsstufe ist so gestaltet, dass an ihrem Ausgang ein Signal abgreifbar ist, das in seinem zeitlichen Verlauf der Einhüllenden des Messsignals oder der positiven oder negativen Halbperiode des Messsignals entspricht.

Bei einer weiteren Ausführungsform der Erfindung sind Steuerungsmittel vorgesehen, die ausgebildet sind, die Auswerteeinheit zum Starten oder Stoppen der Integration eines am Eingang der Auswerteeinheit anliegenden Signals zu veranlassen. Die Steuerungsmittel sind vorzugsweise so gestaltet, daß sie die Auswerteeinheit zur Integration jeweils über die Zeitspanne einer oder einiger Herzperioden veranlassen. Hierzu kann auf bekannte Triggermethoden zurückgegriffen werden. Die Integration wird bei normal schlagendem Herzen an einem bestimmten Phasenpunkt der Herzperiode gestartet, so daß übliche, nicht pathologische Frequenzveränderungen bei der Bestimmung des Integrals des Messsignals nicht ins Gewicht fallen. Die Integrationsdauer, also die vorbestimmte Zeitspanne T, wird also in gewissem Rahmen variabel an die Herzfrequenz angepaßt.

Die Steuerungsmittel sind vorzugsweise jedoch zusätzlich so gestaltet, daß sie die Auswerteeinheit zur Integration über mindestens eine jeweils vorbestimmbare, von der Herzperiodendauer unabhängige Zeitspanne veranlassen können. Dies ist im Falle eines fibrillierenden Herzens erforderlich, da eine Triggerung hier nicht möglich ist. Versagt die Triggerung also, ist dies als erstes Indiz einer Arrhythmie zu werten. Die Vorrichtung reagiert auf diese Situation durch Umstellung auf eine vorbestimmte, feste Zeitspanne für die Integration. Anschließend wird eine neue Impedanzmessung gestartet, um den Zustand des Herzens zu überprüfen.

Bei einer weiteren Ausführungsform der Erfindung weist die Auswerteeinheit einen Speicher auf. Die Auswerteeinheit ist ferner so gestaltet, daß innerhalb der vorbestimmten Zeitspanne das bis dahin maximale und minimale, am Eingang der Auswerteeinheit anliegende Signal fortlaufend bestimmt und in dem Speicher abgelegt werden, daß am Ende der Zeitspanne die Differenz zwischen dem augenblicklich gespeicherten maximalen und minimalen Signal berechnet wird und daß jeweils zu Beginn einer nachfolgenden Zeitspanne die in der vorangegangenen Zeitspanne zuletzt gespeicherten Signale aus dem Speicher gelöscht werden. Der Speicher dient hier also nur zur temporären Aufnahme der augenblicklich als Maximum und Minimum bestimmten Werte. Diese werden überschrieben, sobald innerhalb des Messzeitintervalls ein neues Maximum bzw. Minimum des Messsignals festgestellt wird. Die Bestimmung der Extremwerte kann mit jeder Messzeitspanne neu gestartet werden.

Auch die Zeitspanne, für die die Bestimmung der PPA durchgeführt wird, wird vorzugsweise durch mit der Auswerteeinheit verbundene Steuerungsmittel festgelegt, die so ausgebildet sind, das sie der Auswerteeinheit den Beginn und das Ende der vorbestimmten Zeitspanne signalisieren.

In einer besonders vorteilhaften Ausführungsform sind erfolgt die zeitliche Steuerung de Bestimmung des ersten und des zweiten Signals zentral. Die Steuerungsmittel sind entsprechend so gestaltet, daß sie die Auswerteeinheit zur Integration und Bestimmung der Differenz zwischen dem Maximum und dem Minimum jeweils innerhalb derselben Zeitspanne veranlassen. Auf diese Weise liegen mit dem ersten und zweiten Signal zwei teilweise komplementäre Informationen über die Herztätigkeit innerhalb desselben Zeitraums zur Bewertung vor. Eine Arrhythmie kann zuverlässig und schnell diagnostiziert werden.

In einer weiteren bevorzugten Ausführungsform ist die Auswerteeinheit so gestaltet, daß sie bei Betrieb der Vorrichtung am Ende der jeweiligen Zeitspanne ein solches zweites Signal ausgibt, das dem Integral des Messsignals über die Zeitspanne abzüglich des Produktes der Dauer der Zeitspanne und des Messsignalminimums der Zeitspanne entspricht.

Auf diese Weise kann die Signifikanz des zweiten Signals erhöht werden. Dies wird unmittelbar deutlich, wenn man bedenkt, daß die intrakardiale Impedanz eines fibrillierenden Herzens wegen des wenig veränderlichen Blutvolumens etwa konstant ist und einen Wert annimmt, der geringer als der systolische Impedanzwert ist, jedoch deutlich höher als der diastolische Impedanzwert des Herzens. Das zeitliche Integral $ZI$ der intrakardialen Impedanz des fibrillierenden Herzens wird sich also nur wenig vom dem des normal schlagenden Herzens über die gleiche Zeitspanne T unterscheiden.

Zur Erhöhung der Signifikanz wird also über die Differenz zwischen dem augenblicklichen und einem minimalen, für jede Integrationsperiode T individuellen, minimalen Impedanzwert $Z_0(T)$ integriert. Dieser Wert $Z_0(T)$ wird ohnehin ständig im Rahmen der Bestimmung des PPA festgehalten. Dieser minimale Impedanzwert $Z_0$ entspricht im Normalfall dem diastolischen impedanzsignal. Bezeichnet man also die intrakardiale Impedanz mit Z, das Integrationsintervall mit T und das zweite Signal mit ZI(T), so ist ZI(T) gegeben durch

$$ZI\,(T) = \int_T Z(t)\;dt - Z_0(T) \times T$$

Die Differenzbildung ist der Einfachheit halber, ohne Verlust mathematischer Genauigkeit, der Integration nachgeschaltet und nutzt den in der Integrationszeitspanne T zur Bestimmung der PPA festgehaltenen minimalen Extremwert $Z_0(T)$ der intrakardialen Impedanz Z.

Bei einem fibrillierenden Herzen ist die Differenz zwischen $Z_0(T)$ und Z(t) so gering, daß ZI(T) einen deutlich kleineren Wert annimmt als bei einem normal schlagenden Herzen. Im Zustand der Tachykardie ist zwar eine größere Modulation der intrakardialen Impedanz als bei der Fibrillation nachweisbar, jedoch ist der Wert von ZI(T) noch immer deutlich geringer als beim normal schlagenden Herzen.

Ein weiteres Ausführungsbeispiel der Erfindung weist zur Auswertung des PPAund des ZI-Signals je einen mit einem ersten Speicher für Sollwerte- und/ oder Sollwertintervalle verbundenen Vergleicher auf, der so ausgebildet ist, daß er die Differenz aufeinanderfolgender Extremwerte bzw. das zeitliche Integral des Messsignals mit einem jeweiligen, im ersten Speicher enthaltenen Sollwert oder Sollwertintervall vergleicht und ein Ausgangssignal erzeugt, welches anzeigt, ob das jeweilige Vergleichsergebnis einer Abweichung vom Sollwert oder Sollwertintervall entspricht oder ob nicht und, gegebenenfalls, welche Abweichung vorliegt. Durch die zwei Ausgangssignale der Vergleicher wird einer nachgeschalteten Verarbeitungseinheit also angezeigt, ob das jeweilige Signal im Normbereich liegt oder ob es vom Normbereich abweicht. Bei einer Abweichung ist diesen Ausgangssignalen auch zu entnehmen, um welchen Betrag und in welche Richtung das Messsignal vom jeweiligen Normbereich abweicht.

Die Sollwerte oder Sollwertintervalle, die im ersten Speicher abgelegt sind, können zur weiteren Erhöhung der Zuverlässigkeit des Nachweises tachykarder Herzrhythmusstörungen in gewissem Rahmen adaptiv, d. h. veränderlich sein, beispielsweise entsprechend der augenblicklichen körperlichen Aktivität. Hierzu wird der Speicherinhalt von einer Verarbeitungseinheit beeinflußt, die das Impedanzsignal mit Hilfe eines Algorithmus analysiert, beispielsweise einem "Regional Effective Slope Quality" (RQ-) Algorithmus (vgl. Max Schaldach, Electrotherapy of the Heart, Springer Verlag, Berlin, Heidelberg, New York, 1992, S. 114 ff.). Die Verarbeitungseinheit kann zu diesem Zweck jedoch auch für die Verarbeitung weiterer oder anderer bekannter, die körperliche Aktivität widerspiegelnder Signale ausgebildet sein.

Die bis hierher beschriebenen Vorteile und Merkmale der erfindungsgemäßen Detektionsvorrichtung sind in einer implantierbaren Elektrostimulationsvorrichtung zur Behandlung tachykarder Herzrhythmusstörungen von großem Nutzen.

Günstig ist insbesondere die Integration von Schrittmacherfunktionen in die Therapieeinheit der Elektrostimulationseinrichtung, zusätzlich zur Kardioversion/Defibrillation. Hierfür ist die Therapieeinheit der Elektrostimulationsvorrichtung so gestaltet, daß sie wahlweise auch eine Schrittmacher-Elektrostimulationstherapie an das Herz übertragen kann. Die Schrittmachertherapie kann über dieselbe Elektrode angewandt werden, mit der auch die Impedanz gemessen wird. In diesem Falle erfolgt während der Dauer des Stimulationsimpulses keine Impedanzmessung. Die hierfür erforderliche zeitliche Steuerung der Messeinheit und der Therapieeinheit übernimmt die Steuereinheit.

Bei einer bevorzugten Ausführungsform der erfindungsgemäßen Elektrostiimulationsvorrichtung ist zusätzlich ein mit der Steuereinheit verbundener Signalmusterspeicher vorgesehen, in dem Signalmustern, d. h. Ausgangssignalen oder Kombinationen von Ausgangssignalen der Auswerteeinheit, ein oder mehrere Steuersignale für die Messeinheit, die Auswerteeinheit und/oder die Therapieeinheit zugeordnet sind. Die Steuereinheit ist so gestaltet, daß sie von der Auswerteeinheit her empfangene Ausgangssignale mit den Signalmustern des Signalmusterspeichers vergleicht und die dem jeweils zutreffenden Signalmuster zugeordneten Steuersignale erzeugt und an die entsprechende Einheit übermittelt.

Beispielsweise empfange die Steuereinheit Ausgangssignale von der Auswerteeinheit, die anzeigen, daß der PPA-Wert um 10% unterhalb des zugehörigen Sollwert-Intervalles liegt, während der ZI-Wert nicht vom Sollwert abweicht. Die Steuereinheit vergleicht dieses Signalmuster mit den im Signalmusterspeicher abgelegten und entnimmt dem in diesem Fall zutreffenden Eintrag, daß ein erstes Steuersignal an die Messeinheit zur erneuten Durchführung der Bestimmtung des PPA- und des ZI-Wertes zu senden ist und ein zweites Steuersignal an die Therapieeinheit zur Fortsetzung der normalen Schrittmachertherapie mit den bisher geltenden Parametern. Die Steuereinheit führt diese Schritte aus.

Alternativ oder in Ergänzung zur Steuerung der Elektrostimulationsvorrichtung anhand der Ausgangssignale der Auswerteeinheit mit Hilfe des Signalmusterspeichers kann die Steuereinheit auch auf einen oder mehrere in einem Programmspeicher enthaltene Bewertungsalgorithmen zugreifen, um die Ausgangssignale der Auswerteeinheit zu bewerten und die nötigen Steuersignale zu bestimmen und zu erzeugen. Zur Abarbeitung des Bewertungsalgorithmus ist eine Recheneinheit vorgesehen.

Weitere Vorteile der Erfindung werden bei der nachfolgenden Beschreibung eines Ausführungsbeispiels anhand der Zeichnung deutlich.

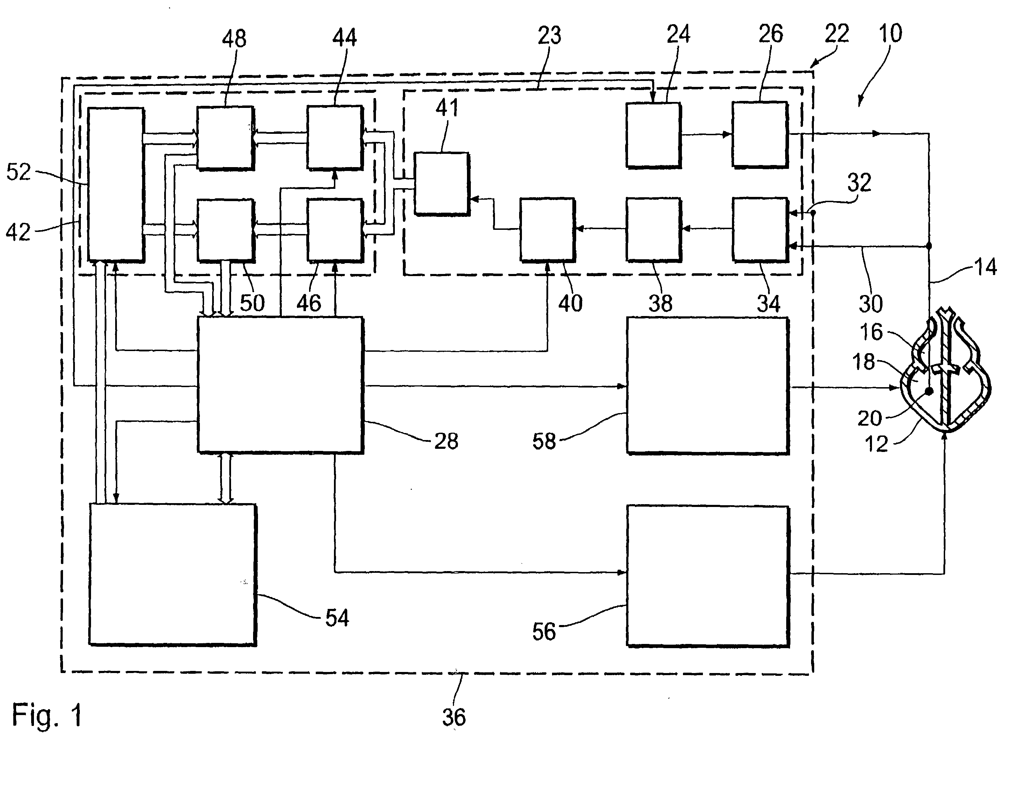

Figur 1 zeigt ein Blockschema einer erfindungsgemäßen Elektrostimulationsvorrichung zu Behandlung tachykarder Herzrhythmusstörungen mit integrierten Schrittmacherfunktionen.

Bei der in Figur 1 dargestellten Stimulationsvorrichtung 10 handelt es sich um einen implantierbaren, integrierten Schrittmacher und Kardioverter/Defibrillator, der zur Stimulation eines menschlischen Herzens 12 eingesetzt wird.

Zur Impedanzmessung ist eine Messelektrode 14 durch den rechten Vorhof 16 in den rechten Ventrikel 18 des Herzens 12 eingeführt. Die Messelektrode 14 weist im Bereich ihres distalen Endes eine Elektrode 20 auf, an der bei der Impedanzmessung ein elektrischer Messstrom in das rechte Ventrikel 18 bzw. das darin enthaltene Blut austritt.

Der Messstrom ist ein elektrischer Wechselstrom mit Rechteck-Pulsform und gleichem Betrag der Stromstärke in beiden Halbperioden. Der Messstrom wird innerhalb eines Aggregats 22 in einer Messeinheit 23 von einer Stromquelle 24 unter Einsatz eines Modulators 26 erzeugt. Die Funktion der Stromquelle 24 wird von einer zentralen Steuerung 28 kontrolliert und mit anderen Funktionsabläufen synchronisiert. Beispielsweise kann die Messelektrode 20 gleichzeitig zur Stimulation des rechten Ventrikels im Rahmen der Schrittmachertherapie verwendet werden. Für die Dauer des Stimulationsimpulses wird dann die Impedanzmessung von der Steuerung 28 gestoppt.

Die Impedanzmessung erfolgt unipolar. Es wird also über entsprechende Signalleitungen 30 und 32 mit Hilfe eines Spannungsrnessers 34 die elektrische Spannung zwischen der Elektrode 20 und dem Gehäuse 36 des Aggregats 22 gemessen. In einem nachgeschalteten Demodulator 38 wird das Spannungssignal von der Modulationsfrequenz bereinigt.

Eine von der Steuerung 28 wahlweise aktivierbare oder deaktivierbare Filterstufen 40 dient zur Reinigung des Messsignals von Frequenzanteilen im Bereich bis etwa 1 Hz (Atemfrequenz). Das gefilterte Messsignal wird anschließend durch einen A/D-Wandler 41 digitalisiert. Die Messeinheit 23 gibt also einen digitalisierten Wert aus, der der gemessenen Spannung entspricht.

Die im Zeitverlauf am Ausgang der Messeinheit 23 ausgegebenen Spannungswerte werden in einer Auswerteeinheit 42 parallel in einem Extremwert-Analysator 44 und in einer Integrations- und Differenzstufe 46 verarbeitet. Beide Auswertestufen 44 und 46 verarbeiten die am Eingang der Auswerteeinheit 42 anliegenden Spannungswerte über gleiche Zeitspannen hinweg. Die Länge dieser Zeitspanne wird jeweils von der Steuerung 28 bestimmt. Diese kann die Zeitspanne auf der Basis vorangeganger Ereignisse, von Berechnungen oder einer Triggerung bestimmen.

Der Extremwert-Analysator 44 vergleicht den aktuell an seinem Eingang anliegenden Spannungswert mit je einem zuvor zwischengespeicherten Minimal- und Maximalwert. Ist der aktuelle Spanungswert größer als der Maximalwert, so wird dieser Spannungswert als neuer Maximalwert in den Zwischenspeicher geschrieben und der alte gelöscht. Ist der aktuelle Spannungswert kleiner als der Minimalwert, so wird dieser Minimalwert vom aktuellen Spannungswert überschrieben. Ist keine der beiden genannten Relationen erfüllt, wird der nächste Spannungswert abgewartet. Auf ein Signal von der Steuereinheit 28 hin wird die Differenz zwischen dem Maximal- und dem Minimalwert gebildet und an einen ersten Vergleicher 48 ausgegeben. Die Zwischenspeicher werden in der Regel für den Beginn der Auswertung des Messsignals einer neuen Messzeitspanne T gelöscht, es sei denn, dass die Steuerung 28 nur eine kurze Unterbrechung der Messung veranlaßt hat.

In der Integrations- und Differenzierstufe 46 werden die nacheinander am Eingang anliegenden Spannungswerte über eine von der Steuerung 28 überwachte und festgelegte Integrationszeitspanne hinweg integriert. Auf ein entsprechendes Signal der Steuerung 28 hin wird die Integration beendet. Die Steuerung 28 veranlaßt den Extremwert-Analysator 44 zur Ausgabe des festgehaltenen Minimalwertes aus dem Zwischenspeicher. Auch hier ist vorgesehen, auf ein entsprechendes Signal der Steuerung 28 hin die Integration nur auszusetzen und auf ein weiteres Steuersignal hin fortzusetzen, ohne daß zwischenzeitlich eine Ausgabe erfolgt. Auf diese Weise kann über mehrere Herzperioden integriert werden, ohne durch einen elektrischen Stimutationsimpuls verursachte Ausschläge des Messsignals aufzunehmen.

Die Ausgangssignale des Extremwertanalysators 44 und der Integrations-/Differenzstufe 46 werden in jeweils einem Vergleicher 48 bzw. 50 mit Sollwerten (oder Sollwertintervallen) verglichen. Hierzu greifen die Vergleicher auf einen Sollwertspeicher 52 zu und bilden die Differenz zwischen dem Ausgangssignal und dem jeweiligen Sollwert (oder den Randwerten des Sollwertintervalls). Die so gebildeten Differenzwerte werden der Steuerung 28 von der Auswerteeinheit 42 als Ergebnisse übermittelt.

Zur Bewertung der Auswertungsergebnisse greift die Steuerung 28 auf einen Signalmusterspeicher 54 zu. Dieser enthält Signalmuster, d.h. Ausgangssignale oder Kombinationen von Ausgangssignalen der Auswerteeinheit. Jedem Signalmuster wird im Signalmusterspeicher ein oder mehrere Steuersignale zugeordnet, das zur situationsgerechten Steuerung der Messeinheit, der Auswerteeinheit und/oder der Therapieeinheit dient. Die Therapieeinheit umfaßt eine Schrittmachereinheit 56 und eine Kardioversions-/Defibrillationseinheit 58.

Die von der Auswerteeinheit gelieferten Ausgangssignale werden mit den Signalmustern des Signalmusterspeichers verglichen. Welche Steuersignale für welche Einheit bei dem jeweils festgestellten Signalmuster zu erzeugen sind, entnimmt die Steuerung dem Signalmusterspeicher 54. Auf diese Weise wird je nach festgestellter Situation die Messung wiederholt oder eine Therapie eingeleitet oder fortgeführt. Eine weitere Rekation auf die Ausgangssignale der Auswerteeinheit 42 kann auch die Anpassung der Sollwertintervalle sein, etwa bei erhöhter körperlicher Aktivität. Hierzu werden die neue Sollwert(intervall)e dem Signalmusterspeicher entnommen und in den Sollwertspeicher 52 geschrieben.

Alternativ oder ergänzend kann auch auf in einem (nicht dargestellten) Programmspeicher abgelegte Bewertungsalgorithmen zugegriffen werden, zu deren Ausführung durch eine (ebenfalls nicht dargestellte) Recheneinheit die Ausgangssignale der Auswerteeinheit als Parameter eingegeben werden. Als Ergebnis der Ausführung eines solchen Bewertungsalgorithmus entnimmt die Steuerung 28 wie oben die zu erzeugenden Steuersignale.

**Patentansprüche**

1. Vorrichtung zur Detektion tachykarder Herzrhythmusstörungen, mit einer Messeinheit (20, 23, 24, 26, 30, 32, 34, 38, 40, 41) zum Aufnehmen und Wiedergeben eines von der intrakardialen Impedanz abhängigen Messsignals an ihrem Ausgang und einer eingangsseitig mit der Messeinheit (20, 23, 24, 26, 30, 32, 34, 38, 40, 41) verbundenen Auswerteeinheit (42, 44, 46, 48, 50, 52), die ausgebildet ist zum Bestimmen und Ausgeben eines ersten Signals, das der Differenz eines maximalen und eines minimalen Messsignals innerhalb mindestens einer definierten Zeitspanne entspricht, **dadurch gekennzeichnet, dass** die Auswerteeinheit (42, 44, 46, 48, 50, 52) zusätzlich ausgebildet ist zum Bestimmen und Ausgeben eines zweiten Signals, das vom Integral des Messsignals über mindestens eine definierte zweite Zeitspanne abhängig ist, und des der zweiten Zeitspanne zugeordneten ersten Signals, wobei die zuerst genannte Zeitspanne vor der zweiten Zeitspanne liegt oder mit der zweiten Zeitspanne identisch ist.

2. Vorrichtung nach Anspruch 1, **dadurch gekennzeichnet, daß** sie Mittel (20, 23, 30, 32, 34, 36) zur unipolaren Impedanzmessung umfaßt.

3. Vorrichtung nach Anspruch 1, **dadurch gekennzeichnet, daß** sie Mittel zur bipolaren Impedanzmessung umfaßt.

4. Vorrichtung nach einem der vorstehenden Ansprüche, **dadurch gekennzeichnet, daß** die Messeinheit mindestens zwei Elektroden (20, 36) aufweist, von denen mindestens eine (20) in eine Herzkammer (16, 18) einführbar ist.

5. Vorrichtung nach einem der vorstehenden Ansprüche, **dadurch gekennzeichnet, dass** sie eine Stromquelle (24) enthält, die mit den Elektroden (20, 36) der Messeinheit (23) derart verbunden ist, daß sie einen vorbestimmten Messstrom zwischen ihnen erzeugt, sowie Spannungsmessmittel (30, 32, 34), die zum Messen einer elektrischen Spannung zwischen den Elektroden (20, 36) mit diesen verbunden sind.

6. Vorrichtung nach einem der Ansprüche 1 bis 4, **dadurch gekennzeichnet, dass** sie eine Spannungsquelle enthält, die mit den Elektroden (20, 36) der Messeinheit derart verbunden ist, daß sie eine vorbestimmte Messspannung zwischen ihnen erzeugt, sowie Strommessmittel, die zum Messen eines elektrischen Stromes zwischen den Elektroden (20, 36) mit diesen verbunden sind.

7. Vorrichtung nach Anspruch 5 oder 6, **dadurch gekennzeichnet, daß** die Spannungs- oder Stromquelle (24) zur Abgabe einer Wechselspannung bzw. eines Wechselstromes mit vorbestimmter Zeitabhängigkeit ausgebildet ist.

8. Vorrichtung nach Anspruch 7, **dadurch gekennzeichnet, daß** sie eine Demodulationseinrichtung (38) aufweist, an deren Eingang im Betrieb der Vorrichtung (10) das durch den von der Stromquelle (24) oder Spannungsquelle erzeugten Wechselstrom bzw. Wechselspannung modulierte Messsignal der Spannungsmessmittel (30, 32, 34) bzw. Strommessmittel anliegt, und die so gestaltet ist, dass an ihrem Ausgang ein Signal abgreifbar ist, das in seinem zeitlichen Verlauf der Einhüllenden des Messsignals oder der positiven oder negativen Halbperiode des Messsignals entspricht.

9. Vorrichtung nach einem der vorstehenden Ansprüche, **gekennzeichnet durch** eine Filterstufe (40) zur Entfernung von Messsignalanteilen mit einer Frequenz bis maximal 1 Hz.

10. Vorrichtung nach einem der vorstehenden Ansprüche, **gekennzeichnet durch** Steuerungsmittel (28), die ausgebildet sind, die Auswerteeinheit (42, 46) zum Starten oder Stoppen der Integration eines am Eingang der Auswerteeinheit (42, 46) anliegenden Signals zu veranlassen.

11. Vorrichtung nach Anspruch 10, **dadurch gekennzeichnet, daß** die Steuerungsmittel (28) so gestaltet sind, daß sie die Auswerteeinheit (42, 46) zur Integration jeweils über die Zeitspanne einer oder einiger Herzperioden veranlassen.

12. Vorrichtung nach Anspruch 10 oder 11, **dadurch gekennzeichnet, daß** die Steuerungsmittel (28) zusätzlich so gestaltet sind, daß sie die Auswerteeinheit (42, 46) zur Integration über mindestens eine jeweils vorbestimmbare, von der Herzperiodendauer unabhängige Zeitspanne veranlassen können.

13. Vorrichtung nach einem der vorstehenden Ansprüche, **dadurch gekennzeichnet, daß** die Auswerteeinheit (42, 44) einen Speicher aufweist.

14. Vorrichtung nach Anspruch 13, **dadurch gekennzeichnet, daß** die Auswerteeinheit (42, 44) so gestaltet ist, daß innerhalb der vorbestimmten Zeitspanne das bis dahin maximale und minimale, am Eingang der Auswerteeinheit (42, 44) anliegende Signal fortlaufend bestimmt und in dem Speicher abgelegt werden, daß am Ende der Zeitspanne die Differenz zwischen dem augenblicklich gespeicherten maximalen und minimalen Signal berechnet wird und daß jeweils zu Beginn einer nachfolgenden Zeitspanne die in der vorangegangenen Zeitspanne zuletzt gespeicherten Signale aus dem Speicher gelöscht werden.

15. Vorrichtung nach Anspruch 14, **gekennzeichnet durch** mit der Auswerteeinheit (42, 44) verbundene Steuerungsmittel (28), die so ausgebildet sind, das sie der Auswerteeinheit (42, 44) den Beginn und das Ende der vorbestimmten Zeitspanne signalisieren.

16. Vorrichtung nach einem der vorstehenden Ansprüche, **dadurch gekennzeichnet, daß** die Steuerungsmittel (28) so gestaltet sind, daß sie die Auswerteeinheit (42, 44, 46) zur Integration und Bestimmung der Differenz zwischen dem Maximum und dem Minimum jeweils innerhalb derselben Zeitspanne veranlassen.

17. Vorrichtung nach einem der vorstehenden Ansprüche, **dadurch gekennzeichnet, daß** die Auswerteeinheit (42, 46) so gestaltet ist, daß sie bei Betrieb der Vorrichtung am Ende der jeweiligen Zeitspanne ein Signal ausgibt, das dem Integral des Messsignals über die Zeitspanne abzüglich des Produktes der Dauer der Zeitspanne und des Messsignalminimums der Zeitspanne entspricht.

18. Vorrichtung nach einem der vorstehenden Ansprüche, **gekennzeichnet durch** je einen Vergleicher (48, 50), der mit einem Sollwerte und/oder Sollwertintervalle enthaltenden Sollwertspeicher (52) verbunden ist und der so ausgebildet ist, daß er bei Betrieb der Vorrichtung die Differenz der Extremwerte bzw. das zeitliche Integral des Messsignals mit einem jeweiligen, im Sollwertspeicher (52) enthaltenen Sollwert oder Sollwertintervall vergleicht und ein Ausgangssignal erzeugt, welches anzeigt, ob das jeweilige Vergleichsergebnis einer Abweichung vom Sollwert oder Sollwertintervall entspricht oder ob nicht und, gegebenenfalls, welche Abweichung vorliegt.

19. Vorrichtung nach einem der vorstehenden Ansprüche, **gekennzeichnet durch** je einen Vergleicher (48, 50), der mit einem Schwankungs-Sollwerte und/oder Schwankungs-Sollwertintervalle enthaltenden Sollwertspeicher (52) verbunden ist und der so ausgebildet ist, daß er bei Betrieb der Vorrichtung die Änderung der Differenz der Extremwerte bzw. des zeitliche Integral des Messsignals zum jeweils vorangehend bestimmten Wert mit einem jeweiligen, im Sollwertspeicher (52) enthaltenen Schwankungs-Sollwert oder Schwankungs-Sollwertintervall vergleicht und ein Ausgangssignal erzeugt, welches anzeigt, ob das jeweilige Vergleichsergebnis einer Abweichung vom Schwankungs-Sollwert oder Schwankungs-Sollwertintervall entspricht oder ob nicht und, gegebenenfalls, welche Abweichung vorliegt.

20. Implantierbare Elektrostimulationsvorrichtung zur Behandlung tachykarder Herzrhythmusstörungen, mit einer Detektionseinheit (20, 23, 30, 32, 42), einer Steuereinheit (28) und einer Therapieeinheit (56, 58), die für die Erzeugung einer an das Herz zu übertragenden Kardioversions- oder Defibrillationselektrotherapie ausgebildet ist, wobei die Detektionseinheit (20, 23, 30, 32, 42) eine Messeinheit (23, 30, 32) zum Aufnehmen und Wiedergeben eines der intrakardialen Impedanz entsprechenden Messsignals an ihrem Ausgang und eine eingangsseitig mit der Messeinheit (23, 30, 32) verbundene Auswerteeinheit (42) aufweist und die Steuereinheit (28) Ausgangssignale der Auswerteeinheit (42) empfängt und die Tätigkeit der Messeinheit (23, 30, 32), der Auswerteeinheit (42) und der Therapieeinheit (56, 58) steuert, **dadurch gekennzeichnet, daß** die Detektionseinheit (20, 23, 30, 32) die Merkmale eines oder mehrerer der Ansprüche 1 bis 18 hat.

21. Elektrostimulationsvorrichtung nach Anspruch 20, **dadurch gekennzeichnet, daß** die Therapieeinheit (56, 58) so gestaltet ist, daß sie wahlweise auch eine Schrittmacher-Elektrostimulationstherapie an das Herz übertragen kann.

22. Vorrichtung nach Anspruch 20 oder 21, **dadurch gekennzeichnet, daß** ein mit der Steuereinheit (28) verbundener Signalmusterspeicher (54) vorgesehen ist, in dem Signalmustern, d.h. Ausgangssignalen oder Kombinationen von Ausgangssignalen der Auswerteeinheit (42), ein oder mehrere Steuersignale für die Messeinheit (23, 24, 40), die Auswerteeinheit (42, 44, 46, 52) und/oder die Therapieeinheit (56, 58) zugeordnet sind, und daß die Steuereinheit (28) so gestaltet ist, daß sie von der Auswerteeinheit (42, 48, 50) her empfangene Ausgangssignale mit den Signalmustern des Signalmusterspeichers (54) vergleicht und die dem jeweils zutreffenden Signalmuster zugeordneten Steuersignale erzeugt und an die entsprechende Einheit übermittelt.

23. Elektrostimulationsvorrichtung nach Anspruch 22, daß die Steuereinheit (28) zur Bestimmung der Steuersignale zusätzlich oder ausschließlich auf einen in einem Programmspeicher enthaltenen Bewertungsafgorithmus zugreift, mit Hilfe dessen in einer Recheneinheit anhand der Ausgangssignale der Auswerteeinheit (42) die zu erzeugenden Steuersignale berechnet werden.

24. Elektrostimulationsvorrichtung nach einem der Ansprüche 20 bis 23, **dadurch gekennzeichnet, daß** sie ein Gehäuse (36) aufweist, das bei der Messung der intrakardialen Impedanz als Elektrode verwendbar ist.

**Claims**

1. Device for detecting tachycardiac rhythm disturbances, comprising a measuring unit (20, 23, 24, 26, 30, 32, 34, 38, 40, 41) for picking up and reproducing a measurement signal dependent on intracardial impedance at its output and an evaluation unit (42, 44, 46, 48, 50, 52) which is connected to the measuring unit (20, 23, 24, 26, 30, 32, 34, 38, 40, 41) at the input side and which is configured to determine and output a first signal which corresponds to the difference of a maximum and a minimum measurement signal within at least one defined period of time, **characterised in that** the evaluation unit (42, 44, 46, 48, 50, 52) is additionally configured to determine and output a second signal, which is dependent on the integral of the measurement signal over at least one defined second period of time, and the first signal associated with the second period of time, the first-mentioned period of time being before the second period of time or identical to the second period of time.

2. Device according to claim 1, **characterised in that** it comprises means (20, 23, 30, 32, 34, 36) for measuring unipolar impedance.

3. Device according to claim 1, **characterised in that** it comprises means for measuring bipolar impedance.

4. Device according to any one of the preceding claims, **characterised in that** the measuring unit comprises at least two electrodes (20, 36), of which at least one (20) can be introduced into a chamber of the heart (16, 18).

5. Device according to any one of the preceding claims, **characterised in that** it contains a current source (24) connected to the electrodes (20, 36) of the measuring unit (23) in such a way that it produces a predetermined measuring current between them, and voltage measuring means (30, 32, 34) connected to the electrodes (20, 36) for measuring an electrical voltage therebetween.

6. Device according to any one of claims 1 to 4, **characterised in that** it contains a voltage source connected to the electrodes (20, 36) of the measuring unit in such a way that it produces a predetermined measuring voltage between them, and current measuring means connected to the electrodes (20, 36) for measuring an electrical current therebetween.

7. Device according to either claim 5 or claim 6, **characterised in that** the voltage or current source (24) is adapted to output an alternating voltage or an alternating current with predetermined time dependency.

8. Device according to claim 7, **characterised in that** it comprises a demodulation device (38), at the input of which, in operation of the device (10), the measurement signal of the voltage measuring means (30, 32, 34) or current measuring means, which is modulated by the alternating current or alternating voltage produced by the current source (24) or voltage source, is applied, the demodulation device being configured such that a signal, which corresponds in its time characteristic to the envelope of the measurement signal or the positive or negative half-period of the measurement signal, can be scanned at its output.

9. Device according to any one of the preceding claims, **characterised by** a filter stage (40) for the removal of measurement signal contents having a frequency of up to 1 Hz.

10. Device according to any one of the preceding claims, **characterised by** control means (28) that are configured to cause the evaluation unit (42, 46) to start or stop integration of a signal at the input of the evaluation unit (42, 46).

11. Device according to claim 10, **characterised in that** the control means (28) are configured so as to cause the evaluation unit (42, 46) to effect integration respectively over the period of time of one or some cardiac periods.

12. Device according to either claim 10 or claim 11, **characterised in that** the control means (28) are also configured so as to cause the evaluation unit (42, 46) to effect integration over at least one respective predeterminable period of time, which is independent of the cardiac period duration.

13. Device according to any one of the preceding claims, **characterised in that** the evaluation unit (42, 44) has a memory.

14. Device according to claim 13, **characterised in that** the evaluation unit (42, 44) is configured such that, within the predetermined period of time, the hitherto maximum and minimum signals at the input of the evaluation unit (42, 44) are continuously determined and stored in the memory, **in that** at the end of the period of time the difference between the currently stored maximum and minimum signals is calculated, and **in that** at the beginning of a respective subsequent period of time the signals last stored in the preceding period of time are erased from the memory.

15. Device according to claim 14, **characterised by** control means (28) that are connected to the evaluation unit (42, 44) and are configured so as to signal to the evaluation unit (42, 44) the beginning and the end of the predetermined period of time.

16. Device according to any one of the preceding claims, **characterised in that** the control means (28) are configured so as to cause the evaluation unit (42, 44, 46) to effect integration and to determine the difference between the maximum and the minimum respectively within the same period of time.

17. Device according to any one of the preceding claims, **characterised in that** the evaluation unit (42, 46) is configured such that, in operation of the device, at the end of the respective period of time it issues a signal which corresponds to the integral of the measurement signal over the period of time less the product of the duration of the period of time and the measurement signal minimum of the period of time.

18. Device according any one of the preceding claims, **characterised by** a respective comparator (48, 50) that is connected to a reference value memory (52) containing reference values and/or reference value ranges and is configured such that, in operation of the device, it compares the difference of the extreme values or the time integral of the measurement signal to a respective reference value or reference value range contained in the reference value memory (52) and produces an output signal which indicates whether or not the respective comparison result corresponds to a deviation from the reference value or reference value range and optionally what deviation is involved.

19. Device according to any one of the preceding claims, **characterised by** a respective comparator (48, 50) that is connected to a reference value memory (52) containing fluctuation reference values and/or fluctuation reference value ranges and is configured such that, in operation of the device, it compares the change in the difference of the extreme values or the time integral of the measurement signal in relation to the respective previously determined value to a respective fluctuation reference value or fluctuation reference value range contained in the reference value memory (52) and produces an output signal which indicates whether or not the respective comparison result corresponds to a deviation from the fluctuation reference value or fluctuation reference value range and optionally what deviation is involved.

20. Implantable electrostimulation device for the treatment of tachycardiac rhythm disturbances, comprising a detection unit (20, 23, 30, 32, 42), a control unit (28) and a therapy unit (56, 58) that is configured to produce a cardioversion or defibrillation electrotherapy to be applied to the heart, the detection unit (20, 23, 30, 32, 42) comprising a measuring unit (23, 30, 32) for receiving and reproducing a measurement signal corresponding to the intracardial impedance at its output and an evaluation unit (42) connected on the input side to the measuring unit (23, 30, 32), and the control unit (28) receiving output signals from the evaluation unit (42) and controlling the activity of the measuring unit (23, 30, 32), the evaluation unit (42) and the therapy unit (56, 58), **characterised in that** the detection unit (20, 23, 30, 32) has the features of one or more of claims 1 to 18.

21. Electrostimulation device according to claim 20, **characterised in that** the therapy unit (56, 58) is configured such that it can also selectively apply a pacemaker electrostimulation therapy to the heart.

22. Device according to either claim 20 or 21, **characterised in that** a signal pattern memory (54) connected to the control unit (28) is provided, in which one or more signals for controlling the measuring unit (23, 24, 40), the evaluation unit (42, 44, 46, 52) and/or the therapy unit (56, 58) are associated with signal patterns, i.e. output signals or combinations of output signals from the evaluation unit (42), and **in that** the control unit (28) is configured such that it compares output signals received from the evaluation unit (42, 48, 50) to the signal patterns of the signal pattern memory (54) and produces the control signals associated with the respectively correct signal pattern and transmits them to the corresponding unit.

**23.** Electrostimulation device according to claim 22, **characterised in that**, for determining the control signals, the control unit (28) additionally or exclusively accesses an assessment algorithm that is contained in a program memory and by means of which the control signals to be produced are calculated in a computing unit on the basis of the output signals of the evaluation unit (42).

**24.** Electrostimulation device according to any one of claims 20 to 23, **characterised in that** it comprises a housing (36) which can be used as an electrode in the measurement of intracardial impedance.

**Revendications**

**1.** Dispositif pour détecter des perturbations du rythme cardiaque par tachycardie, comportant une unité de mesure (20,23,24,26,30,32,34,38,40,41) pour enregistrer et reproduire un signal de mesure, qui dépend de l'impédance intracardiaque, sur sa sortie et une unité d'évaluation (42,44,46,48,50,52) qui est reliée côté entrée à l'unité de mesure (20,23,24,26,30,32,34,38,40,41) et qui est agencée de manière à déterminer et délivrer un premier signal, qui correspond à la différence d'un signal de mesure maximum et d'un signal de mesure minimum à l'intérieur d'au moins un intervalle de temps défini, **caractérisé en ce que** l'unité d'évaluation (42,44,46,48,50,52) est agencée en outre de manière à déterminer et délivrer un second signal, qui dépend de l'intégrale du signal de mesure pendant au moins un second intervalle de temps défini, et le premier signal associé au second intervalle de temps, l'intervalle de temps indiqué en premier étant situé en avant du second intervalle de temps ou étant identique au second intervalle de temps.

**2.** Dispositif selon la revendication 1, **caractérisé en ce qu'**il comprend des moyens (20,23,30,32,34,36) pour réaliser la mesure unipolaire d'impédance.

**3.** Dispositif selon la revendication 1, **caractérisé en ce qu'**il comprend des moyens pour réaliser la mesure bipolaire d'impédance.

**4.** Dispositif selon l'une des revendications précédentes, **caractérisé en ce que** l'unité de mesure comporte au moins deux électrodes (20,36), dont l'une au moins (20) peut être introduite dans une chambre (16,18) du coeur.

**5.** Dispositif selon l'une des revendications précédentes, **caractérisé en ce qu'**il contient une source de courant (24), qui est reliée aux électrodes (20,36) de l'unité de mesure (23) de telle sorte qu'il produit un courant de mesure prédéterminé entre ces électrodes, ainsi que des moyens de mesure de tension (30,32,34) qui, pour la mesure d'une tension électrique entre les électrodes (20,36), sont reliés à ces dernières.

**6.** Dispositif selon l'une des revendications 1 à 4, **caractérisé en ce qu'**il contient une source de tension qui est reliée aux électrodes (20,36) de l'unité de mesure de telle sorte qu'elle produit une tension de mesure prédéterminée entre elles, ainsi que des moyens de mesure de courant qui, pour la mesure d'un courant électrique entre les électrodes (20,36), sont reliés à ces dernières.

**7.** Dispositif selon la revendication 5 ou 6, **caractérisé en ce que** la source de tension ou de courant (24) est conçue pour la délivrance d'une tension alternative ou d'un courant alternatif avec une dépendance prédéterminée vis-à-vis du temps.

**8.** Dispositif selon la revendication 7, **caractérisé en ce qu'**il comporte un dispositif de démodulation (38), à l'entrée duquel, lors du fonctionnement du dispositif (10), le signal de mesure des moyens de mesure de tension (30,32,34) ou des moyens de mesure de courant, qui est modulé par le courant alternatif ou la tension alternative produit par la source de courant (24) ou par la source de tension, et que le dispositif de démodulation est agencé de telle sorte que sur sa sortie peut être prélevé un signal, dont la variation correspond à l'enveloppe du signal de mesure ou à l'alternance positive ou négative du signal de mesure.

**9.** Dispositif selon l'une des revendications précédentes, **caractérisé par** un étage de filtre (40) pour produire des composantes de signaux de mesure ayant une fréquence atteignant au maximum jusqu'à 1 Hz.

**10.** Dispositif selon l'une des revendications précédentes, **caractérisé par** des moyens de commande (28) qui sont orientés de manière à amener l'unité d'évaluation (42, 46) à faire démarrer ou à arrêter l'intégration d'un signal appliqué à l'entrée de l'unité d'évaluation (42,46).

**11.** Dispositif selon la revendication 10, **caractérisé en ce que** les moyens de commande (28) sont agencés de telle sorte qu'ils amènent l'unité d'évaluation (42,46) à effectuer l'intégration respectivement pendant l'intervalle de temps ou pendant quelques périodes du coeur.

**12.** Dispositif selon la revendication 10 ou 11, **caractérisé en ce que** les moyens de commande (28) sont agencés en outre de telle sorte qu'ils peuvent amener l'unité d'évaluation (42,46) à réaliser une intégration sur au moins un intervalle de temps pouvant être respectivement prédéterminé, qui est indépendant de la durée de la période cardiaque.

**13.** Dispositif selon l'une des revendications précédentes, **caractérisé en ce que** l'unité d'évaluation (42,44) comporte une mémoire.

**14.** Dispositif selon la revendication 13, **caractérisé en ce que** l'unité d'évaluation (42,44) est agencée de telle sorte que pendant l'intervalle de temps prédéterminé, les signaux jusqu'alors maximum et minimum, qui sont présents à l'entrée de l'unité d'évaluation (42,44), sont déterminés de façon continue et sont mémorisés dans la mémoire, qu'à la fin de l'intervalle de temps, la différence entre les signaux maximum et minimum mémorisés instantanément est calculée et que respectivement au début d'un intervalle de temps suivant, les signaux, mémorisés en dernier lieu dans l'intervalle de temps précédent, sont effacés de la mémoire.

**15.** Dispositif selon la revendication 14, **caractérisé par** les moyens de commande (28), qui sont reliés à l'unité d'évaluation (42,44) et sont agencés de telle sorte qu'ils signalent à l'unité d'évaluation (42,44) le début et la fin de l'intervalle de temps prédéterminé.

**16.** Dispositif selon l'une des revendications précédentes, **caractérisé en ce que** les moyens de commande (28) sont agencés de telle sorte qu'ils amènent l'unité d'évaluation (42,44,46) à intégrer et déterminer la différence entre le maximum et le minimum respectivement pendant le même intervalle de temps.

**17.** Dispositif selon l'une des revendications précédentes, **caractérisé en ce que** l'unité d'évaluation (42,46) est agencée de telle sorte que lors du fonctionnement du dispositif, elle délivre à la fin de l'intervalle de temps respectif un signal qui correspond à l'intégrale du signal de mesure pendant l'intervalle de temps, diminuée du produit de la durée de l'intervalle de temps par le minimum du signal de mesure de l'intervalle de temps.

**18.** Dispositif selon l'une des revendications précédentes, **caractérisé par** un comparateur respectif (48,50), qui est relié à une mémoire de valeurs de consigne (52) qui contient des valeurs de consigne et/ou des intervalles de valeurs de consigne et qui est agencé de telle sorte que lors du fonctionnement du dispositif, il compare la différence des valeurs extrêmes ou de l'intégrale dans le temps du signal de mesure à une valeur de consigne ou à un intervalle de valeurs de consigne respectif, contenu dans la mémoire de valeurs de consigne (52), et produit un signal de sortie qui indique si le résultat respectif de la comparaison correspond à un écart de la valeur de consigne ou de l'intervalle de valeurs de consigne ou non et, éventuellement, quel écart est présent.

**19.** Dispositif selon l'une des revendications précédentes, **caractérisé par** un comparateur respectif (48,50) qui est relié à une mémoire de valeurs de consigne contenant des valeurs de consigne de variation et/ou des intervalles de valeurs de consigne de variation et est agencé de telle sorte que lors du fonctionnement du dispositif, il compare la variation de la différence des valeurs extrêmes ou de l'intégrale dans le temps du signal de mesure pour la valeur respectivement déterminée précédemment à une valeur de consigne respective de variation, ou à un intervalle respectif de valeurs de consigne de variation, contenu dans la mémoire de valeurs de consigne (52) et produit un signal de sortie qui indique si le résultat respectif de la comparaison correspond à un écart par rapport à la valeur de consigne de variation ou par rapport à l'intervalle de valeurs de consigne de variation ou non et éventuellement quel écart est présent.

**20.** Dispositif d'électrostimulation implantable pour le traitement de perturbations du rythme cardiaque par tachycardie, comportant une unité de détection (20,30,32, 42), une unité de commande (28) et une unité de thérapie (56,58) qui est agencée de manière à produire une électrothérapie de cardioversion ou de défibrillation devant être transmise au coeur, et dans lequel l'unité de détection (20,23,30,32, 42) comporte l'unité de mesure (23,30,32) pour enregistrer et reproduire un signal de mesure correspondant à l'impédance intracardiaque, au niveau de sa sortie et une unité d'évaluation (42) reliée côté entrée à l'unité de mesure (23,30,32), et l'unité de commande (28) reçoit des signaux de sortie de l'unité d'évaluation (42) et commande l'activité de l'unité de mesure (23,30,32), -de l'unité d'évaluation (42) et de l'unité de thérapie (56, 58), **caractérisé en ce que** l'unité de détection (20,23,30, 32) présente les caractéristiques d'une ou de plusieurs des revendications 1 à 18.

**21.** Dispositif d'électrostimulation selon la revendication 22, **caractérisé en ce que** l'unité de thérapie (56,58) est agencée de telle sorte qu'au choix elle peut transmettre au coeur également une thérapie d'électrostimulation de stimulateur.

**22.** Dispositif selon la revendication 20 ou 21, **caractérisé en ce qu'**il est prévu une mémoire (54) de modèles de signaux, qui est reliée à l'unité de commande (28) et dans laquelle un ou plusieurs signaux de commande pour l'unité de mesure (23,24,40), l'unité d'évaluation (42,44,46,52) et/ou l'unité de thérapie (56,58), sont associés à des modèles de signaux, c'est-à-dire des signaux de sortie ou des combinaisons de signaux de sortie de l'unité d'évaluation (42), et que l'unité de commande (28) est agencée de telle sorte qu'elle compare les signaux de sortie, reçus en provenance de l'unité d'évaluation (42,46, 50), aux modèles de signaux de la mémoire de modèles de signaux (54) et produit les signaux de commande associés au modèle de signaux respectivement concerné, et les transmet à l'unité correspondante.

23. Dispositif d'électrostimulation selon la revendication 22, **caractérisé en ce que** pour la détermination des signaux de commande l'unité de commande (28) accède en supplément ou exclusivement à un algorithme d'évaluation contenu dans une mémoire de programmes et à l'aide duquel les signaux de commande devant être produits sont calculés dans une unité de calcul sur la base des signaux de sortie de l'unité d'évaluation (42).

24. Dispositif d'électrostimulation selon l'une des revendications 20 à 23, **caractérisé en ce qu'**il comporte un boîtier (36) qui peut être utilisé en tant qu'électrode lors de la mesure de l'impédance intracardiaque.

Fig. 1